# EUROPEAN PATENT SPECIFICATION

(11) **EP 2 743 260 B1**
(45) Date of publication and mention of the grant of the patent: **19.10.2016**
(21) Application number: 12822639.6
(22) Date of filing: 24.04.2012
(51) Int. Cl.: C07D 207/273

(54) **CRYSTAL FORM II OF (S)-4-HYDROXY-2-OXO-1-PYRROLIDINE ACETAMIDE AND PREPARATION METHOD THEREOF**
KRISTALLFORM II VON (S)-4-HYDROXY-2-OXO-1-PYRROLIDIN-ACETAMID UND HERSTELLUNGSVERFAHREN DAVON
FORME CRISTALLINE II DU (S)-4-HYDROXY-2-OXO-1-PYRROLIDINE-ACÉTAMIDE ET PROCÉDÉ DE PRÉPARATION DE CELLE-CI

(30) Priority: 11.08.2011 CN 201110230244
(43) Date of publication of application: 18.06.2014
(73) Proprietor: CHONGQING RUNZE PHARMACEUTICAL CO., LTD., Yubei District Chongqing 401120 (CN)
(72) Inventor: YE, Lei, Yubei Chongqing 401120 (CN)
(74) Representative: Høiberg A/S
(86) International application number: PCT/CN2012/074582
(87) International publication number: WO 2013/020391

(56) References cited:
- EP-A1- 2 573 070
- EP-A1- 2 743 258
- EP-A1- 2 743 259
- CN-A- 1 956 953
- CN-A- 101 367 757
- CN-A- 101 575 309
- CN-A- 102 101 836
- CN-A- 103 342 673
- S. BANFI ET AL: "Cyclic GABA-GABOB analogues IV - Activity on learning and memory", FARMACO, EDIZIONE SCIENTIFICA, vol. 39, no. 1, 1984, pages 16-22, XP001008330, ISSN: 0430-0920
- Guiliano Bandoli ET AL: "Solid-state structure and conformation of the nootropic agent 4-hydroxy-2-oxo-1-pyrrolidineacetamide: X-ray and theoretical self-consistent field molecular orbital (SCF-MO) studies", CHEMICAL & PHARMACEUTICAL BULLETIN, vol. 33, no. 10, 1 October 1985 (1985-10-01), pages 4395-4401, XP055153439, ISSN: 0009-2363

## Description

### Field of the Invention

The present invention relates to a drug of crystal form II of (S)-4-hydroxy-2-oxo-1-pyrrolidine acetamide and, more particularly, relates to a crystal form II of (S)-4-hydroxy-2-oxo-1-pyrrolidine acetamide and a preparation method thereof.

### Description of the Prior art

Oxiracetam is a nootropic drug, which is firstly synthesized by Italy Smith Kline Beecham Ltd in 1974 and came into the market in 1987 in Italy. (S)-oxiracetam is a single enantiomer of oxiracetam, the chemical name of which is (S)-4-hydroxy-2-oxo-1-pyrrolidine acetamide (hereinafter referred to as (S)-oxiracetam or (S)-olaxiracetam for short).

Oxiracetam can promote the synthesis of phosphoryl choline and phosphoryl ethanolamine, and promote cerebral metabolism, shows stimulatory effects to specific central nervous pathway through blood-brain barrier, increases the ATP/ADP ratio in the brain and increases the synthesis of protein and nucleic acid in the brain, can improve learning and memory functions of patients with intelligence disorder, and the drug itself have no direct vascular activity, nor the central excitation, its influence on learning and memory abilities is a persistent promoting effect.

CN1513836, CN1948285 and CN101121688 respectively reports a process for synthesis of racemate of two isomers (S)-oxiracetam and (R)-oxiracetam; CN101367757 and CN101575309 respectively reports a method of the preparation of (S)-oxiracetam; CN1424034, CN1555794, CN1562000 and CN101152175 respectively reports a method of the preparation of a injection formulation of oxiracetam, a dispersible tablet of oxiracetam, a lyophilized formulation of oxiracetam and a novel formulation of oxiracetam; WO93/06826 discloses that (S)-oxiracetam has good therapeutic effects on the improvement of intelligence.

Il Farmaco Ed. Sc. (1984), vol.39, p.16-22, and WO 2011/143872, published on 24.11.2011, describe the crystallisation of (S)-oxiracetam from acetone/water.

### Summary of the Invention

It is an objective of the present invention to provide a crystal form II of (S)-olaxiracetam, which is in high purity and has good medicinal therapeutic effects on improving intelligence.

A crystal form of (S)-olaxiracetam in the present invention is named as a crystal form II of (S)-olaxiracetam by the present inventors.

A further objective of the present invention is to provide a preparation method of the crystal form II of (S)-olaxiracetam. The preparation method with low cost is simple and the product (S)-olaxiracetam obtained by this method contains low content of impurity and has high purity.

The technical solutions of the present invention are as follows:
The crystal form II of (S)-olaxiracetam is characterized by having diffraction peaks appearing at the following 2θ diffraction angles: 10.669, 13.25, 13.847, 14.198, 16.729, 17.934, 18.746, 18.816, 20.273, 20.413, 21.431, 21.617, 21.663, 23.38, 24.324, 24.415, 26.069, 26.107, 27.901, 28.621, 28.925, 29.449, 29.484, 31.702, 36.516, 37.685 and39.721.

The crystal form II of (S)-olaxiracetam is characterized by having an X-ray powder diffraction data expressed in terms of d (Å)-values and relative intensity percentages I (%) as shown below:

| d value | I value | d value | I value |
|---|---|---|---|
| 8.2857 | 9 | 6.6765 | 11 |
| 5.2953 | 13 | 4.9422 | 54 |
| 4.7298 | 33 | 4.7123 | 14 |
| 4.3769 | 47 | 4.3471 | 58 |
| 4.1429 | 100 | 4.1077 | 11 |
| 4.099 | 34 | 3.8018 | 9 |
| 3.6563 | 11 | 3.6429 | 44 |
| 3.4154 | 26 | 3.4104 | 40 |
| 3.1951 | 14 | 3.1164 | 16 |
| 3.0843 | 13 | 3.0306 | 19 |
| 3.0271 | 9 | 2.8202 | 26 |

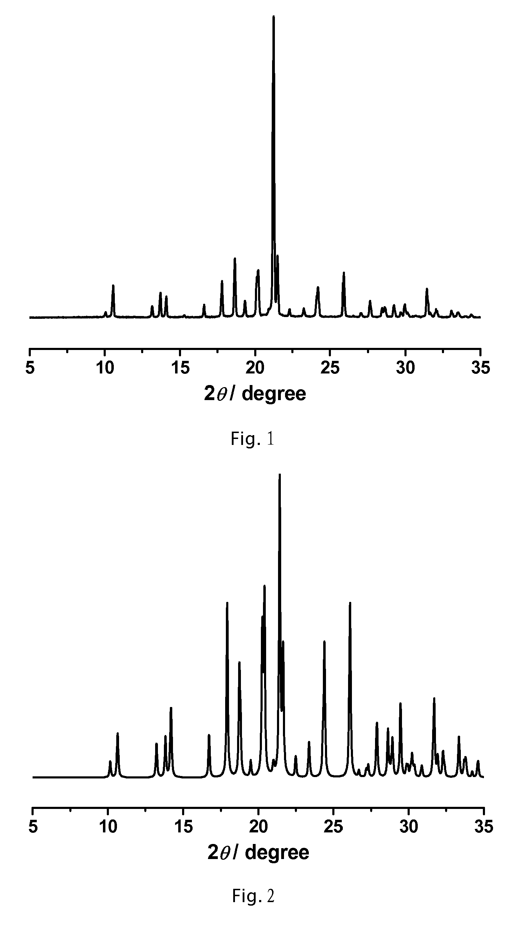

The crystal form II of (S)-olaxiracetam is characterized by having an X-ray powder diffraction pattern shown in Figure 1.

The infrared spectrum produced by the crystal form II of (S)-olaxiracetam in the present invention exhibits absorption peaks appearing at the following waven umbers:
3318 (cm⁻¹), 3223 (cm⁻¹), 2929 (cm⁻¹), 2875 (cm⁻¹), 1680 (cm⁻¹), 1487 (cm⁻¹), 1402 (cm⁻¹), 1276 (cm⁻¹), 1220 (cm⁻¹), 1078 (cm⁻¹), 968 (cm⁻¹), 943 (cm⁻¹), 694 (cm⁻¹) and 611 (cm⁻¹).

The preparation method of the crystal form II of (S)-olaxiracetam is characterized by comprising the following steps:
(1). dissolving crude (S)-olaxiracetam of purity ≤ 92 wt % in water to form a solution, the solution is allowed to stand at 5∼10°C for 1∼3 days, and colorless transparent crystals precipitate; the mass to volume ratio (g/ml) of the crude (S)-oxiracetam to water is 1:0.7∼1;
(2). the colorless transparent crystals precipitated above being filtered and washed with ice water; the mass to volume ratio (g/ml) of the colorless transparent crystals to ice water is 1: 1∼2 and the temperature of the ice water is 0∼5°C;
(3). drying under vacuum to obtain the crystal form II of (S)-oxiracetam hydrate.

The present invention has the following advantages:
The crystal form II of (S)-olaxiracetam in the present invention has high purity which can reach 99% and has good therapeutic effects when used in drugs for improving intelligence. The preparation method of the crystal form II of (S)-olaxiracetam, with mild control conditions and low cost, is simple and suitable for large-scale industrialization production. The obtained crystal form of (S)-olaxiracetam has high purity (the purity of the crude crystal form II of (S)-olaxiracetam increases from 92% to 99%). Moreover, the preparation method of the present invention can be very determined to obtain the crystal form II of (S)-oxiracetam hydrate in good reproducibility.

### Brief Description of the Drawings

Figure 1 is an X-ray powder diffraction pattern of the crystal form II of (S)-oxiracetam hydrate.
Figure 2 is an X-ray powder diffraction pattern of the crystal form of (S)-oxiracetam simulated from the crystal structure data.
Figure 3 is a single crystal structure of the crystal form II of (S)-oxiracetam hydrate.
Figure 4 is a crystal cell packing view of the crystal form II of (S)-oxiracetam hydrate.
Figure 5 is a differential scanning calorimetry (DSC) thermogram of the crystal form II of (S)-oxiracetam hydrate.
Figure 6 is a mass spectrum (EI-MS) of the crystal form II of (S)-oxiracetam hydrate.
Figure 7 is a Raman spectrum of the crystal form II of (S)-oxiracetam hydrate.
Figure 8 is a thermogravimetric analysis (TG) curve of the crystal form II of (S)-oxiracetam hydrate.
Figure 9 is an infrared (IR) spectrum of the crystal form II of (S)-oxiracetam hydrate.

### Detailed Description of the Preferred Embodiments

### Example 1

A preparation method of (S)-oxiracetam is as follows:
1g of crude (S)-oxiracetam (of 92% purity) was dissolved in 0.8ml of water to form a solution, then the solution was allowed to stand at 7-8 °C for 1-3 days, and colorless transparent crystals precipitated. The colorless transparent crystals were filtered, washed with 1.5ml of ice water (1-2°C) and dried under vacuum at room temperature, to afford 0.7g of colorless crystals in 99% purity.

### Example 2

A preparation method of (S)-oxiracetam is as follows:
1g of crude (S)-oxiracetam (of 90% purity) was dissolved in 1ml of water to form a solution, then the solution was allowed to stand at 5 °C for 2-3 days, and colorless transparent crystals precipitated. The colorless transparent crystals were filtrated, washed with 1ml of ice water (5°C) and dried under vacuum at room temperature, to afford 0.6g of colorless crystals in 99% purity.

The crude (S)-oxiracetam was synthesized according to the following steps:
(a) 518.4g of glycinamide hydrochloride, 394g of sodium bicarbonate and 3.7L of anhydrous ethanol were charged into a three-necked reaction flask to form a mixture. The PH value of the mixture was maintained at 7.4 or thereabouts. Then the mixture was heated to reflux with stirring;
(b) 781.6g of ethyl (S) 4-chloro-3-hydroxybutyrate was added into the solution drops after refluxing for 2h. During the dropwise addition, the remaining 394g of sodium bicarbonate was added in 8 portions, and the amount of the base added in each portion was controlled by detecting the PH value of the reaction mixture to maintain the pH≤8.5;
(c) The reaction mixture was further refluxed for 24h after the dropwise addition of ethyl (S) 4-chloro-3-hydroxybutyrate was completed. The reaction was terminated until the content of (S)-4-hydroxy-2-oxo-1-pyrrolidine acetamide measured by HPLC was 75%. The obtained solution was thermal filtered and concentrated to obtain crude (S)-4-hydroxy-2-oxo-1-pyrrolidine acetamide;
(d) The obtained crude product was dissolved in 800mL of water to obtain a solution, the solution obtained was treated with 8.0L of 001x7 strong acid styrene type cation exchange resin and the fractions containing products were collected. The collected aqueous solution was neutralized with 201x7 strong base styrene type anion exchange resin. The neutralization was determined as completed until the measured pH value of the solution reached 7.0±0.1.

### Example 3 crystal parameter determination of crystal form of (S)-oxiracetam

The X-ray powder diffraction pattern of the crystal form of (S)-oxiracetam hydrate obtained in example 1 was determined using a Bruker D8 Advance diffractometer, and determination conditions is as follows: Cu Kα, 40kV, light source 40mV, step size 0.018°, scanning speed 4°/min, scanning range 5∼40°, room temperature. In the X-ray Powder Diffraction pattern of the crystal form of (S)-oxiracetam hydrate, diffraction peaks were appearing at the following 2θ diffraction angles: 10.669, 13.25, 13.847, 14.198, 16.729, 17.934, 18.746, 18.816, 20.273, 20.413, 21.431, 21.617, 21.663, 23.38, 24.324, 24.415, 26.069, 26.107, 27.901, 28.621, 28.925, 29.449, 29.484, 31.702, 36.516, 37.685 and 39.721. The X-ray powder diffraction pattern is shown in Figure 1.

The X-ray powder diffraction pattern of the crystal form of (S)-olaxiracetam hydrate is expressed in terms of interplanar spacing d, Bragg angle (2θ) and relative intensity percentages I as shown below:

| 2θ / degree | d / Å | relative intensity *I* / % |
|---|---|---|
| 10.669 | 8.2857 | 9 |
| 13.25 | 6.6765 | 11 |
| 13.847 | 6.3903 | 13 |
| 14.198 | 6.2329 | 21 |
| 16.729 | 5.2953 | 13 |
| 17.934 | 4.9422 | 54 |
| 18.746 | 4.7298 | 33 |
| 18.816 | 4.7123 | 14 |
| 20.273 | 4.3769 | 47 |
| 20.413 | 4.3471 | 58 |
| 21.431 | 4.1429 | 100 |
| 21.617 | 4.1077 | 11 |
| 21.663 | 4.099 | 34 |
| 23.38 | 3.8018 | 9 |
| 24.324 | 3.6563 | 11 |
| 24.415 | 3.6429 | 44 |
| 26.069 | 3.4154 | 26 |
| 26.107 | 3.4104 | 40 |
| 27.901 | 3.1951 | 14 |
| 28.621 | 3.1164 | 16 |
| 28.925 | 3.0843 | 13 |
| 29.449 | 3.0306 | 19 |
| 29.484 | 3.0271 | 9 |
| 31.702 | 2.8202 | 26 |

The crystal structure of (S)-oxiracetam hydrate obtained in example 1 of the present invention was determined at 150(2) K using a crystal diffractometer Oxford Gemini S Ultra with graphite monochromator. Cu Kα ray (1.54178Å) was used and data was collected in ω/2 scanning mode. Data reduction and absorption correction were performed with the software package of Rigaku RAPID AUTO (Rigaku, 1998, Ver2.30). Space group was determined according to the systematic absence law and was verified by refinement data. All the crystal structures were solved using SHELXS-97 (direct methods) and refined using SHELXL-97 programs (full-matrix least-squares) and hydrogen atomic coordinate was added by theory calculation. Crystal parameters of the crystal form of (S)-olaxiracetam hydrate are shown in the table below:

**Table of crystallographic parameters of the crystal form of (S)-olaxiracetam hydrate**

| | |
|---|---|
| formula | C₁₂H₂₂N₄O₇ |
| formula weight | 334.34 |
| temperature | 150(2) K |
| wavelength | 1.54178 Å (Cu Kα) |
| crystal system, space group | orthorhombic, *P*2(1) 2 (1) 2 (1) |
| cell parameter | a = 9.4245(3) Å, α=90° |
| | b = 9.4597 (3) Å, β = 90° |
| | c = 17.3882 (6) Å, γ= 90° |
| cell volume | 1550.21 (9) Å³ |
| Z value, theoretical density | 4, 1.433 g/cm³ |
| linear absorption coefficient | 1.010 mm⁻¹ |
| structure factor F (000) | 712 |
| crystal size | 0.10 * 0.14 * 0.10 mm |
| range for date collection θ | 5.09 ∼ 62.53° |
| range of crystal plane index | -10 <= h <= 9, -9 <= k <= 9, -14 <= 1<=19 |
| reflections collected /unique | 4524/2269 [Rint = 0.0265] |
| intact rate (θ= 26.00°) | 98.9% |
| absorption correction method | Ψ-scanning |
| maximum and minimum transmittance | 0.9058 / 0.8799 |
| refinement method | full-matrix least-squares on F² |
| unique reflections data / restrains / parameters | 2421 / 0 / 208 |
| goodness-of-fit index GOOF value | 1.095 |
| R indices [I>2σ (I)]* | R₁ = 0.0298, wR₂ = 0.0697 |
| R indices (all data) | R₁= 0.0338, wR₂ = 0.0730 |
| largest difference peak / hole | 0.146 / -0.146 e • A³ |

| | |
|---|---|
| **R*₁=∑\| \|*F*₀\|-\|*F*_{c}\| \|/∑\|*F*₀\|, w*R*₂=[∑w(*F₀²-F_{c}²*)²/∑w(*F₀*²)²]^{1/2}, w=[σ²(*F*₀)²+(0.1(max(0, *F*₀²) +2 *F_{c}²*)/3)²]⁻¹ | |

Figure 1 is an X-ray powder diffraction (PXRD) pattern of (S)-oxiracetam hydrate in the present invention.

Figure 2 is an X-ray powder diffraction pattern of the crystal form of (S)-oxiracetam hydrate simulated from the crystal structure data. The peaks in Figure 1 coincide with those in Figure 2, which indicates that the obtained crystal form of (S)-oxiracetam hydrate is a pure single crystal form.

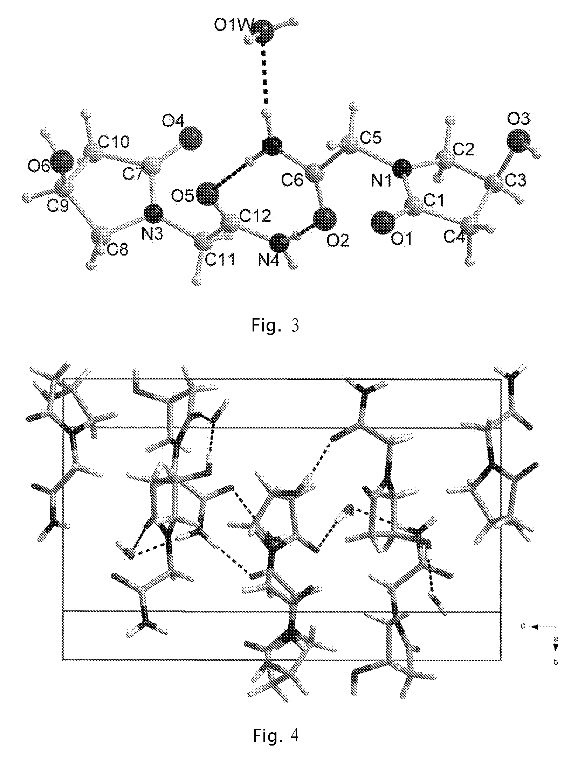

The single crystal structure of the crystal form of (S)-oxiracetam hydrate is shown in Figure 3.

The crystal cell packing view of the crystal form of (S)-oxiracetam hydrate is shown in Figure 4.

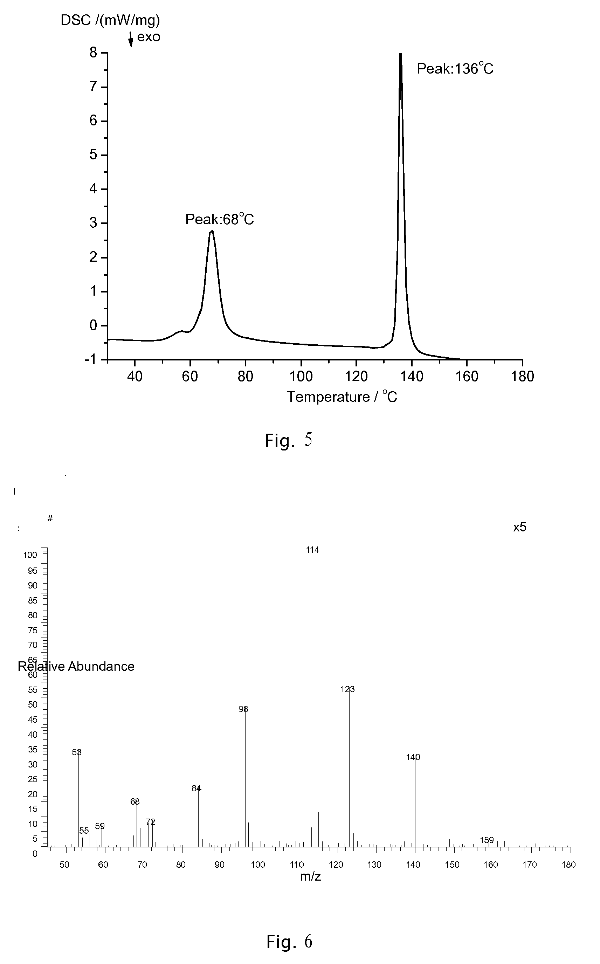

The differential scanning calorimetry (DSC) thermogram of the crystal form of (S)-oxiracetam hydrate is shown in Figure 5 where the thermal transition temperatures are 68 and 136°C.

The mass spectrum of the crystal form of (S)-oxiracetam hydrate is shown in Figure 6.

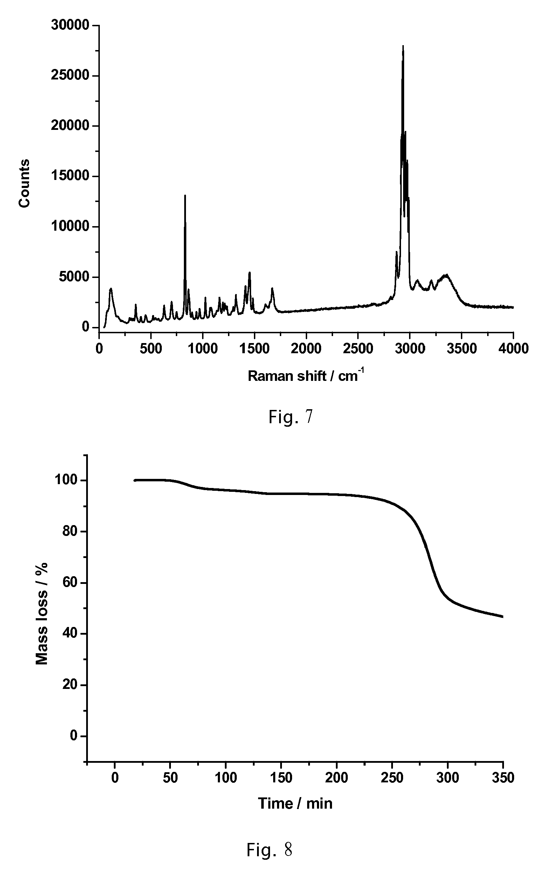

The Raman spectrum of the crystal form of (S)-oxiracetam hydrate is shown in Figure 7.

The thermogravimetric analysis curve of the crystal form of (S)-oxiracetam hydrate is shown in Figure 8.

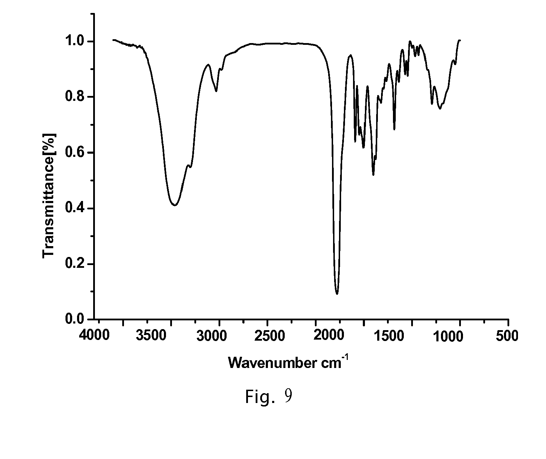

The infrared spectrum of the crystal form of (S)-oxiracetam hydrate is shown in Figure 9, which exhibits absorption peaks appearing at the following wavenumbers: 3318, 3223, 2929, 2875, 1680, 1487, 1402, 1276, 1220, 1078, 968, 943, 694 and 611.

The X-ray powder diffraction pattern of example 2 is the same as that of example 1 after detection. Therefore, the preparation method of the present invention is in good reproducibility which can obtain stable crystal form of (S)-oxiracetam hydrate.

## Claims

1. A crystal form II of (S)-4-hydroxy-2-oxo-1-pyrrolidine acetamide, **characterized by** having diffraction peaks appearing at the following 2θ diffraction angles: 10.669, 13.25, 13.847, 14.198, 16.729, 17.934, 18.746, 18.816, 20.273, 20.413, 21.431, 21.617, 21.663, 23.38, 24.324, 24.415, 26.069, 26.107, 27.901, 28.621, 28.925, 29.449, 29.484, 31.702, 36.516, 37.685 and 39.721.

2. The crystal form II of (S)-4-hydroxy-2-oxo-1-pyrrolidine acetamide according to claim 1, **characterized by** having an X-ray powder diffraction data expressed in terms of d (Å)-values and relative intensity percentages I (%) as shown below:
| d value | I value | d value | I value |
|---|---|---|---|
| 8.2857 | 9 | 6.6765 | 11 |
| 5.2953 | 13 | 4.9422 | 54 |
| 4.7298 | 33 | 4.7123 | 14 |
| 4.3769 | 47 | 4.3471 | 58 |
| 4.1429 | 100 | 4.1077 | 11 |
| 4.099 | 34 | 3.8018 | 9 |
| 3.6563 | 11 | 3.6429 | 44 |
| 3.4154 | 26 | 3.4104 | 40 |
| 3.1951 | 14 | 3.1164 | 16 |
| 3.0843 | 13 | 3.0306 | 19 |
| 3.0271 | 9 | 2.8202 | 26 |

3. The crystal form II of (S)-4-hydroxy-2-oxo-1-pyrrolidine acetamide, **characterized by** having an X-ray powder diffraction (PXRD) pattern as shown in Figure 1.

4. A preparation method of the crystal form II of (S)-4-hydroxy-2-oxo-1-pyrrolidine acetamide according to any one of claims 1-3, **characterized by** comprising the following steps:
(1). dissolving crude (S)-4-hydroxy-2-oxo-1-pyrrolidine acetamide of purity ≤ 92 wt % in water to form a solution, wherein the solution is allowed to stand at about 5 to 10°C for 1 to 3 days, and colorless transparent crystals precipitate; the mass to volume ratio (g/ml) of the crude (S)-oxiracetam to water being ranging from 1: 0.7 to 1:1;
(2). filtering the colorless transparent crystals precipitated above and washing the crystals with ice water; wherein the mass to volume ratio (g/ml) of the colorless transparent crystal to ice water is ranging from 1: 1 to 1:2 and the temperature of the ice water is 0 to 5°C;
(3). drying the crystals under vacuum to obtain the crystal form II of (S)-4-hydroxy-2-oxo-1-pyrrolidine acetamide.

## Patentansprüche

1. Kristallform II von (S)-4-Hydroxy-2-oxo-1-pyrrolidinacetamid, **gekennzeichnet durch** das Aufweisen von Beugungspeaks, die an den folgenden 2θ-Beugungswinkeln auftreten: 10,669, 13,25, 13,847, 14,198, 16,729, 17,934, 18,746, 18,816, 20,273, 20,413, 21,431, 21,617, 21,663, 23,38, 24,324, 24,415, 26,069, 26,107, 27,901, 28,621, 28,925, 29,449, 29,484, 31,702, 36,516, 37,685 und 39,721.

2. Kristallform II von (S)-4-Hydroxy-2-oxo-1-pyrrolidinacetamid gemäß Anspruch 1, **gekennzeichnet durch** das Aufweisen von Röntgenpulverbeugungsdaten, ausgedrückt als d (Å)-Werte und Prozentanteile der relativen Intensität I (%), wie unten angegeben:
| d-Wert | I-Wert | d-Wert | I-Wert |
|---|---|---|---|
| 8,2857 | 9 | 6,6765 | 11 |
| 5,2953 | 13 | 4,9422 | 54 |
| 4,7298 | 33 | 4,7123 | 14 |
| 4,3769 | 47 | 4,3471 | 58 |
| 4,1429 | 100 | 4,1077 | 11 |
| 4,099 | 34 | 3,8018 | 9 |
| 3,6563 | 11 | 3,6429 | 44 |
| 3,4154 | 26 | 3,4104 | 40 |
| 3,1951 | 14 | 3,1164 | 16 |
| 3,0843 | 13 | 3,0306 | 19 |
| 3,0271 | 9 | 2,8202 | 26 |

3. Kristallform II von (S)-4-Hydroxy-2-oxo-1-pyrrolidinacetamid, **gekennzeichnet durch** das Aufweisen eines Röntgenpulverbeugungs(PXRD)-Musters wie in Figur 1 dargestellt.

4. Herstellungsverfahren der Kristallform II von (S)-4-Hydroxy-2-oxo-1-pyrrolidinacetamid gemäß einem der Ansprüche 1-3,
**gekennzeichnet durch** das Umfassen der folgenden Schritte:
(1). Auflösen von rohem (S)-4-Hydroxy-2-oxo-1-pyrrolidinacetamid mit einer Reinheit ≤92 Gew.-% in Wasser zur Bildung einer Lösung, wobei die Lösung 1 bis 3 Tage bei etwa 5 bis 10 °C stehen gelassen wird und farblose, transparente Kristalle ausfallen; wobei das Masse-zu-Volumen-Verhältnis (g/ml) des rohen (S)-Oxiracetams zu Wasser im Bereich von 1:0,7 bis 1:1 ist;
(2). Abfiltrieren der oben ausgefällten, farblosen, transparenten Kristalle und Waschen der Kristalle mit Eiswasser;
wobei das Masse-zu-Volumen-Verhältnis (g/ml) des farblosen, transparenten Kristalls zu Eiswasser im Bereich von 1:1 bis 1:2 liegt und die Temperatur des Eiswassers 0 bis 5 °C beträgt;
(3). Trocknen der Kristalle unter Vakuum, um die Kristallform II von (S)-4-Hydroxy-2-oxo-1-pyrrolidinacetamid zu erhalten.

## Revendications

1. Forme cristalline II du (S)-4-hydroxy-2-oxo-1-pyrrolidine-acétamide, **caractérisée par le fait qu'**elle présente des pics de diffraction apparaissant aux angles de diffraction 2θ suivants : 10,669, 13,25, 13,847, 14,198, 16,729, 17,934, 18,746, 18,816, 20,273, 20,413, 21,431, 21,617, 21,663, 23,38, 24,324, 24,415, 26,069, 26,107, 27,901, 28,621, 28,925, 29,449, 29,484, 31,702, 36,516, 37,685 et 39,721.

2. Forme cristalline II du (S)-4-hydroxy-2-oxo-1-pyrrolidine-acétamide selon la revendication 1, **caractérisée par le fait qu'**elle présente des données de diffraction des rayons X sur poudre exprimées en termes de valeurs d (Å) et de pourcentages d'intensité relative I (%) tels qu'indiqués ci-dessous :
| valeur d | valeur I | valeur d | valeur I |
|---|---|---|---|
| 8,2857 | 9 | 6,6765 | 11 |
| 5,2953 | 13 | 4,9422 | 54 |
| 4,7298 | 33 | 4,7123 | 14 |
| 4,3769 | 47 | 4,3471 | 58 |
| 4,1429 | 100 | 4,1077 | 11 |
| 4,099 | 34 | 3,8018 | 9 |
| 3,6563 | 11 | 3,6429 | 44 |
| 3,4154 | 26 | 3,4104 | 40 |
| 3,1951 | 14 | 3,1164 | 16 |
| 3,0843 | 13 | 3,0306 | 19 |
| 3,0271 | 9 | 2,8202 | 26 |

3. Forme cristalline II du (S)-4-hydroxy-2-oxo-1-pyrrolidine-acétamide, **caractérisée par le fait qu'**elle présente un spectre de diffraction des rayons X sur poudre (PXRD) tel qu'illustré sur la Figure 1.

4. Procédé de préparation de la forme cristalline II du (S)-4-hydroxy-2-oxo-1-pyrrolidine-acétamide selon l'une quelconque des revendications 1 à 3,
**caractérisé par le fait qu'**il comprend les étapes suivantes :
(1). dissolution de (S)-4-hydroxy-2-oxo-1-pyrrolidine-acétamide brut de pureté ≤ 92 % en poids dans l'eau pour former une solution, dans lequel on laisse la solution reposer à environ 5 à 10 °C pendant 1 à 3 jours, et des cristaux transparents incolores précipitent ; le rapport de la masse au volume (g/ml) du (S)-oxiracétame brut à l'eau étant situé dans la plage de 1/0,7 à 1/1 ;
(2). filtration des cristaux transparents incolores précipités ci-dessus et lavage des cristaux avec de l'eau glacée ; dans lequel le rapport de la masse au volume (g/ml) des cristaux transparents incolores à l'eau glacée est situé dans la plage de 1/1 à 1/2 et la température de l'eau glacée est de 0 à 5 °C ;
(3). séchage des cristaux sous vide pour obtenir la forme cristalline II du (S)-4-hydroxy-2-oxo-1-pyrrolidine-acétamide.
